(19) Europäisches Patentamt
European Patent Office
Office européen des brevets

(11) **EP 3 389 761 B1**

(12) **FASCICULE DE BREVET EUROPEEN**

(45) Date de publication et mention
de la délivrance du brevet:
**13.10.2021 Bulletin 2021/41**

(51) Int Cl.:
***A61N 1/05*** *(2006.01)*

(21) Numéro de dépôt: **16810353.9**

(86) Numéro de dépôt international:
**PCT/EP2016/080683**

(22) Date de dépôt: **12.12.2016**

(87) Numéro de publication internationale:
**WO 2017/102662 (22.06.2017 Gazette 2017/25)**

(54) **SONDE IMPLANTABLE COMPRENANT UN MANCHON, NOTAMMENT POUR LA STIMULATION D'UN NERF, ET MÉTHODE DE FABRICATION DE CE MANCHON**

IMPLANTIERBARE SONDE MIT HÜLSE, INSBESONDERE FÜR DIE STIMULATION EINES NERVS, UND VERFAHREN ZUR HERSTELLUNG DER BESAGTEN HÜLSE

IMPLANTABLE PROBE COMPRISING A SLEEVE, PARTICULARLY FOR THE STIMULATION OF A NERVE, AND METHODS FOR PRODUCING SAID SLEEVE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.12.2015 FR 1562848**
**18.12.2015 FR 1562853**

(43) Date de publication de la demande:
**24.10.2018 Bulletin 2018/43**

(73) Titulaire: **Sorin CRM SAS**
**92140 Clamart (FR)**

(72) Inventeurs:
• **BEFAHY, Stéphane**
**1082 Bruxelles (BE)**
• **MEVEL, Hervé**
**1450 Chastre (St-Géry) (BE)**
• **CALLEGARI, Vincent**
**1435 Corbais (BE)**

(74) Mandataire: **Grünecker Patent- und**
**Rechtsanwälte**
**PartG mbB**
**Leopoldstraße 4**
**80802 München (DE)**

(56) Documents cités:
**EP-A1- 2 959 938    WO-A1-2014/106023**
**US-A- 4 602 624    US-A1- 2008 004 673**

## Description

L'invention concerne les "dispositifs médicaux implantables actifs" tels que définis par la directive 90/385/CEE du 20 juin 1990 du Conseil des communautés européennes, plus précisément les implants permettant la stimulation d'un organe de forme cylindrique allongée, et/ou le recueil de potentiels électriques sur un tel organe.

L'invention concerne plus particulièrement la stimulation des nerfs, spécialement la stimulation du nerf vague dans le cas de thérapies dites VNS (*Vagus Nerve Stimulation)*.

Toutefois, cette application ne présente aucun caractère limitatif, l'invention pouvant être utilisée pour la stimulation/détection de tout autre organe, ou encore à d'autres fins telles que la délivrance locale d'un agent actif, etc. à cet organe, dès lors que l'organe cible présente une forme cylindrique allongée.

La stimulation du système nerveux est une thérapie reconnue à l'égard de très nombreux troubles tels que l'épilepsie, la douleur, l'insuffisance cardiaque, l'apnée, l'obésité, etc.

Les dispositifs utilisés à cet effet comprennent une sonde pourvue d'une électrode implantée sur le nerf vague et un générateur délivrant des impulsions électriques sur cette électrode.

La thérapie VNS consiste à générer des salves d'impulsions répétitives, synchronisées ou non sur le rythme cardiaque selon le trouble que l'on souhaite traiter, ces impulsions se superposant aux signaux naturellement véhiculés par le système nerveux, organisé en boucle fermée. La stimulation du nerf vague pourra agir en efférence, directement vers un organe, ou en afférence, vers le cerveau pour influer sur le système nerveux central : le signal arbitraire constitué par les impulsions VNS sera alors interprété par le système nerveux central comme une sollicitation que ce dernier va tenter de compenser pour s'y opposer, et empêcher de produire ainsi les effets attendus.

L'invention concerne plus particulièrement le dispositif implanté à l'interface électrode/nerf, qui permet de maintenir les électrodes au contact du nerf ou au voisinage étroit de celui-ci.

Compte tenu de la configuration allongée et approximativement cylindrique d'un nerf périphérique tel que le nerf vague, le dispositif le plus couramment utilisé se présente sous la forme d'un manchon de forme tubulaire, enroulé autour du nerf. Le manchon est généralement réalisé en un élastomère tel que le silicone, du fait de l'excellente biocompatibilité de ce matériau, et il porte sur sa face intérieure, appliquée contre le nerf, les électrodes de stimulation (et/ou de détection).

Un tel manchon est par exemple divulgué par le US 4 602 624 A. Le manchon décrit dans ce document est réalisé à partir de deux feuilles d'élastomère laminées ensemble, l'une d'entre elles ayant été étirée au préalable dans une direction privilégiée de précontrainte. La feuille composite résultante est ensuite découpée pour donner une pièce rectangulaire qui, du fait de la précontrainte de l'une des feuilles, aura naturellement tendance, à l'état libre, à s'enrouler en spirale sur elle-même autour d'un axe perpendiculaire à la direction de précontrainte (une "spirale" étant une courbe plane s'enroulant régulièrement autour d'un point dont elle s'écarte de plus en plus).

Par rapport à un manchon rigide, le manchon décrit dans ce document présente l'avantage d'une simplicité de mise en oeuvre : il suffit au chirurgien de le dérouler, de le passer sous le nerf puis de le relâcher pour qu'il vienne de lui-même s'enrouler autour du nerf. De plus, ce manchon est auto-adaptable : en effet, immédiatement après l'implantation un processus inflammatoire normal produit un gonflement temporaire du nerf, qui disparait ensuite. Si l'on choisit un manchon spiralé souple avec un diamètre intérieur au repos légèrement inférieur au diamètre du nerf, ce manchon - avec ses électrodes - restera toujours étroitement plaqué contre le nerf même si le diamètre de celui-ci varie, et sans risque de compression excessive risquant de détériorer irréversiblement les tissus nerveux.

Ce dispositif n'est cependant pas dépourvu d'inconvénients.

Un *premier inconvénient* se présente au moment de l'implantation : pour poser le manchon, après avoir atteint le nerf cible le chirurgien tire le nerf hors de l'incision qu'il a pratiquée, pour pouvoir glisser le manchon déroulé à l'endroit choisi. Pendant cette manoeuvre, la traction exercée sur le nerf peut entrainer localement, aux extrémités du manchon, des contraintes relativement élevées sur les tissus nerveux, susceptibles d'endommager ces derniers. Une autre cause possible d'endommagement du nerf est la durée de la procédure, qui peut exposer le nerf à l'air pendant trop longtemps. Il est donc nécessaire que la procédure d'implantation du manchon soit très brève, en limitant autant que possible les manipulations du nerf. Également pendant l'implantation, les coins du manchon ont tendance à s'enrouler d'eux-mêmes et gênent l'implantation, ce qui complique la tâche du chirurgien.

Un *deuxième inconvénient,* qui apparait après l'implantation, tient au fait que le bord le plus intérieur du manchon, c'est-à-dire le bord enroulé autour du nerf, vient appuyer contre ce dernier et exerce le long de la ligne de contact une pression ayant tendance à contraindre, voire à déformer, le nerf, avec des effets potentiellement délétères. La rigidité des manchons peut aussi provoquer à leurs extrémités des forces de cisaillement inacceptables. Le risque existe aussi que lors de l'implantation le chirurgien ait laissé s'enrouler en sens inverse la partie du bord externe du manchon, qui forme alors une seconde spire en sens inverse de la première. Le rayon de courbure n'est alors plus celui qui était prévu, avec pour conséquence un risque de surépaisseur.

Un *troisième inconvénient* est lié au processus de fabrication. Comme indiqué plus haut, le manchon est réalisé en laminant ensemble deux feuilles d'élastomère, avec une précontrainte directionnelle appliquée à l'une d'entre elles. Ces feuilles étant très minces (leur épaisseur typique est d'environ 100 μm), des problèmes d'homogénéité du matériau et de tolérance de l'épaisseur peuvent apparaitre sur l'étendue de la surface d'une même feuille aussi bien qu'entre deux feuilles, ce qui limite la reproductibilité du procédé de production des manchons. Il est certes possible de pallier cet inconvénient en utilisant des feuilles de grande dimension, mais avec une incidence négative sur le processus industriel. Il est également possible d'utiliser des feuilles plus épaisses, mieux contrôlables au cours du processus, mais avec un risque accru d'endommagement du nerf du fait d'une moins grande souplesse et donc d'une moindre aptitude du manchon à se conformer à la morphologie du nerf dans la zone d'implantation.

Enfin, un *quatrième inconvénient* réside du fait de l'utilisation d'un matériel isolant comme support des électrodes et de la terminaison franche des extrémités du manchon, il en résulte la création d'électrodes virtuelles de type anode ou cathode responsables d'effets de blocage ou de stimulations non souhaitées.

Il est également connu du US 5 251 634 A un manchon pour une sonde implantable composée d'une pluralité d'hélices. Une paire de rubans conducteurs électriques est incorporée dans le matériau respectivement de deux hélices. D'autres hélices formant le manchon sont utilisées pour réaliser le maintien du manchon sur le nerf.

Cette solution technique présente également plusieurs inconvénients. Notamment, une implantation d'un tel dispositif ayant une pluralité d'hélices est difficile à mettre en place autour du nerf. De plus, les courants de stimulation à appliquer sur le nerf ne sont pas appliqués uniquement sur ce dernier car aucune protection continue sur ce manchon n'est prévue. Enfin, un manchon à hélices n'est pas adapté à recevoir des électrodes devant avoir plusieurs points de contact, notamment pour une stimulation tripolaire.

D'autres manchons pour une sonde implantable ont également été conçus notamment de forme cylindrique avec une ouverture longitudinale permettant l'implantation du manchon autour du nerf. Différents systèmes de fermeture du manchon ont alors été conçus. Toutefois, ces manchons sont de diamètre fixe et afin de ne pas abimer le nerf lors de l'implantation du manchon, celui-ci doit être choisi d'une taille supérieure au diamètre du nerf. Une bonne liaison entre le manchon et le nerf est donc impossible et par conséquent, les électrodes ne sont pas maintenues en contact avec le nerf. WO-A-2014/106023, US-A-2008/004673 et EP-A-2959938 divulguent des sondes implantables.

Le besoin subsiste donc d'un manchon auto-enroulable en élastomère mince, pouvant être produit par un procédé industriel performant, avec un degré élevé de reproductibilité, tout en supprimant les effets secondaires connus des manchons existants.

Le but de l'invention est de résoudre ces problèmes, en proposant une nouvelle structure de manchon auto-enroulable en spirale :

- qui facilite une implantation rapide par le chirurgien, sans introduire de contraintes excessives sur le nerf, notamment qui évite de provoquer une force de cisaillement sur les bords du manchon ;
- qui respecte l'intégrité anatomique du nerf après implantation, tout en continuant d'assurer un maintien satisfaisant du manchon au site d'implantation choisi ;
- qui puisse être produit par un procédé optimisé du point de vue des contraintes industrielles ; et
- qui supprime la génération d'électrodes virtuelles.

À cet effet, l'invention propose une sonde implantable comprenant un manchon auto-enroulable tel que décrit notamment par le US 4 602 624 A précité, c'est-à-dire comprenant, de manière en elle-même connue, un manchon apte à être enroulé autour d'un organe de forme cylindrique allongée tel qu'un nerf, et comprenant une feuille en matériau élastiquement déformable portant au moins une électrode de détection/stimulation. La feuille est précontrainte de manière à permettre son auto-enroulement, depuis une position initiale où la feuille est maintenue sous contrainte à l'état déployé, jusqu'à une position finale où la feuille est librement enroulée en spirale en formant un manchon autour de l'organe. La feuille est délimitée par un bord latéral extérieur du manchon après enroulement, un bord latéral intérieur du manchon après enroulement, et un premier bord transversal réunissant des premières extrémités homologues du premier bord latéral et du second bord latéral, et un second bord transversal opposé réunissant des secondes extrémités homologues du premier bord latéral et du second bord latéral.

De façon caractéristique de l'invention, dans la position finale du manchon, la feuille comprend au moins une zone ayant une contrainte à proximité du premier et/ou du second bord transversal sur les bords longitudinaux inférieure à la contrainte d'une zone située dans une région médiane de la feuille de sorte à créer au moins une extrémité de la feuille évasée.

Selon diverses caractéristiques subsidiaires avantageuses :

- la zone ayant une plus faible contrainte est localisée dans au moins une zone s'étendant jusqu'à 50 % de la largeur de la feuille à proximité du premier et/ou du second bord transversal, la largeur de la feuille étant définie par la distance entre le premier et le second bord transversal ;
- la zone de plus faible contrainte présente un gradient de contrainte décroissant sur la largeur de la zone, s'étendant de l'intérieur du manchon vers le bord transversal du manchon ;
- la zone ayant une plus faible contrainte est formée d'une feuille d'élastomère et la zone située en zone médiane comprend au moins deux feuilles d'élastomère ; et/ou

- la zone de plus faible contrainte présente un gradient de contrainte décroissant sur la largeur de la zone, s'étendant de l'intérieur du manchon vers le bord transversal du manchon.

L'invention concerne également une méthode de fabrication d'un manchon pour une sonde implantable, apte à être enroulé autour d'un organe de forme cylindrique allongée tel qu'un nerf, ce manchon comprenant une feuille en matériau élastiquement déformable portant au moins une électrode de détection/stimulation, la feuille étant précontrainte de manière à permettre son auto-enroulement depuis une position initiale où la feuille est maintenue sous contrainte à l'état déployé jusqu'à une position finale où la feuille est librement enroulée en spirale en formant un manchon autour de l'organe, la feuille étant délimitée par un bord latéral extérieur du manchon après enroulement, un bord latéral intérieur du manchon après enroulement, et un premier bord transversal réunissant des premières extrémités homologues du premier bord latéral et du second bord latéral, et un second bord transversal opposé réunissant des secondes extrémités homologues du premier bord latéral et du second bord latéral, la feuille étant formée d'au moins une première et une seconde feuille d'élastomère.

Dans un premier mode de mise en œuvre, la méthode comprend une étape d'étirement de la deuxième feuille d'élastomère dans une direction perpendiculaire à l'axe d'enroulement de la feuille, un étirement plus faible étant réalisé dans une zone à proximité du premier et/ou du second bord transversal sur les bords longitudinaux par rapport à une zone précontrainte située dans une région médiane de la feuille, et une étape de fixation de la deuxième feuille d'élastomère étirée sur la première feuille d'élastomère.

De préférence, la zone ayant une plus faible contrainte est localisée dans au moins une zone s'étendant jusqu'à 30 % de la largeur de la feuille d'élastomère à proximité du premier et/ou du second bord transversal, la largeur de la feuille d'élastomère étant définie par la distance entre le premier et le second bord transversal de la feuille.

Avantageusement, la zone de plus faible contrainte présente un gradient de contrainte décroissant sur la largeur de la zone, s'étendant de l'extrémité de la zone la plus proche de la partie médiane de la feuille d'élastomère vers le bord transversal de la zone de plus faible précontrainte. Dans un second mode de mise en œuvre, la méthode comprend une étape d'étirement de la deuxième feuille d'élastomère dans une direction perpendiculaire à l'axe d'enroulement de la feuille, une étape de fixation de la deuxième feuille d'élastomère étirée sur la première feuille d'élastomère, la première feuille d'élastomère et la seconde feuille d'élastomère étant de largeurs différentes, la largeur étant définie par la distance entre les bords latéraux desdites feuilles d'élastomère.

Selon diverses caractéristiques subsidiaires avantageuses :

- l'étape de fixation consiste à fixer la feuille d'élastomère de plus faible largeur sensiblement centrée sur la largeur de la feuille d'élastomère la plus large ;
- la feuille d'élastomère de plus faible largeur a une largeur comprise entre 60 et 80 % de la largeur de l'autre feuille d'élastomère ;
- la première feuille d'élastomère est de largeur inférieure à la seconde feuille d'élastomère ; et/ou
- la seconde feuille d'élastomère est de largeur inférieure à la première feuille d'élastomère.

On va maintenant décrire un exemple de mise en œuvre de la présente invention, en référence aux dessins annexés où les mêmes références désignent d'une figure à l'autre des éléments identiques ou fonctionnellement semblables.

La Figure 1 est une vue générale de présentation d'un ensemble de stimulation VNS, montrant le générateur et le nerf vague ainsi que la sonde utilisée.
La Figure 2 est une vue d'un manchon selon l'état de la technique, enroulé autour du nerf vague pendant la procédure d'implantation.
La Figure 3 est une vue en plan du manchon selon l'invention, en configuration déroulée telle que ce manchon se présente au moment de sa fabrication.
La Figure 4 est une section dans le sens de l'épaisseur du manchon de la

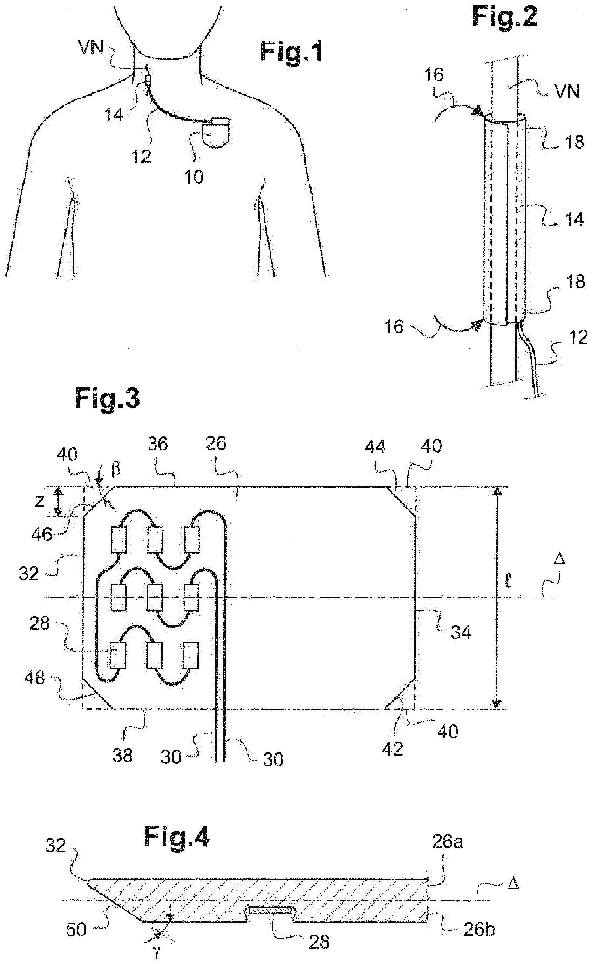

Figure 3, en vue partielle dans la région de l'un des bords, montrant notamment la structure en deux couches superposées laminées ensemble.

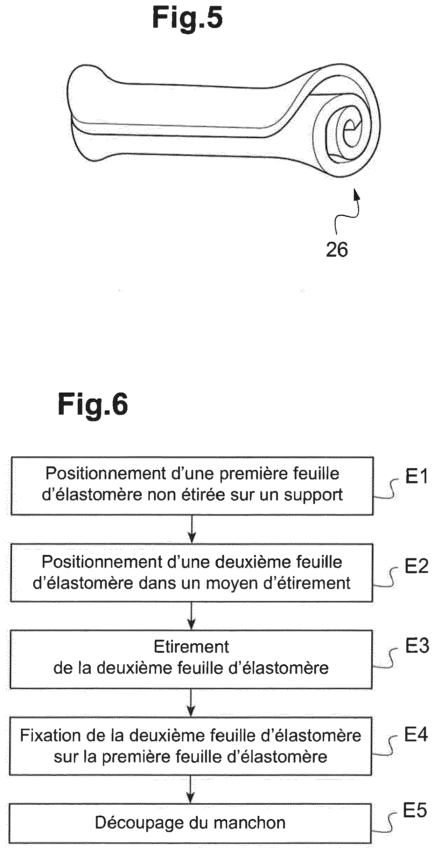

La Figure 5 est une vue en perspective du manchon de la Figure 3 dans sa configuration libre, enroulé sur lui-même, conformément à l'invention.
La Figure 6 est une méthode de fabrication d'un manchon conformément à l'invention.

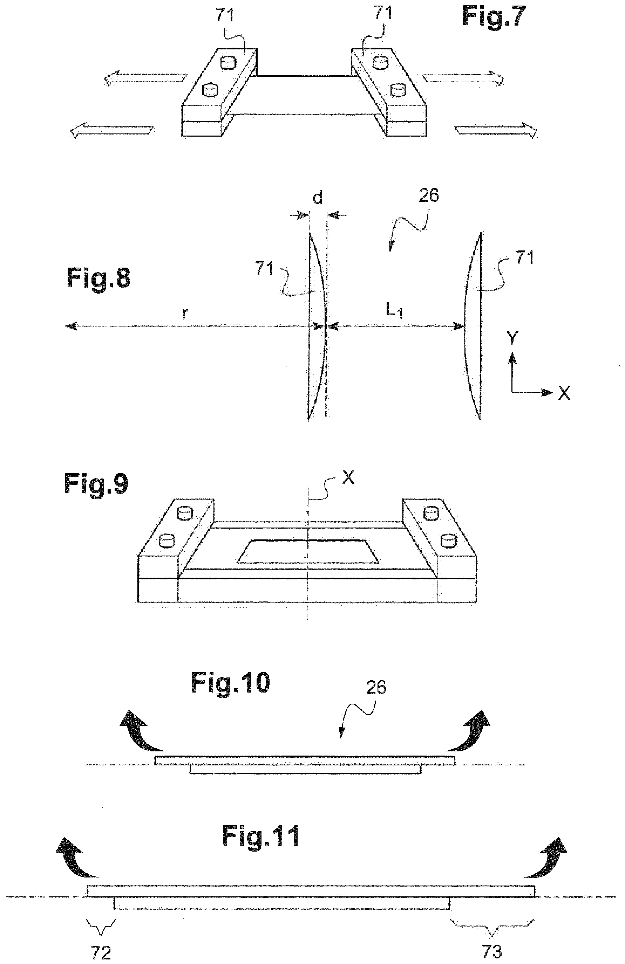

La Figure 7 est une illustration de moyens d'étirement d'une feuille conformément à l'invention.
La Figure 8 illustre des moyens d'étirement courbes de forme circulaire du côté de la feuille d'élastomère.
La Figure 9 illustre une variante de mise en œuvre de la méthode d'obtention d'une feuille ayant des gradients de précontrainte conformément à l'invention.
La Figure 10 illustre un manchon formé de deux feuilles d'élastomère de largeurs différentes, pour la variante de la Figure 9.
La Figure 11 illustre un manchon formé de deux feuilles d'élastomère de largeurs différentes non centrées sur leur largeur, pour la variante de la Figure 9.

On va maintenant décrire un exemple de réalisation d'une sonde de stimulation du nerf vague selon l'invention, cet exemple n'étant aucunement limitatif comme on l'a indiqué en introduction.

Sur la Figure 1, la référence 10 désigne le boitier d'un générateur implantable de stimulation VNS. Les impulsions de stimulation générées sont délivrées par une sonde 12 portant à sa partie distale un manchon 14 pourvu d'électrodes à proximité du nerf vague VN et susceptible de stimuler celui-ci par les salves d'impulsions produites par le générateur 10.

La Figure 2 est une vue d'un manchon 14 selon l'état de la technique, enroulé autour du nerf vague VN pendant la procédure d'implantation.

Pour poser le manchon, le chirurgien a dû isoler le nerf vague VN, pour pouvoir y glisser le manchon déroulé à l'endroit choisi. Après auto-enroulement en spirale, le manchon 14 se présente comme illustré sur la Figure 2. Dans cette configuration particulière, des contraintes sont exercées par le manchon cylindrique dans la région 16 du fait de la discontinuité de rigidité entre la partie du nerf enfermée à l'intérieur du manchon 14, et la partie libre au-delà du manchon. Cette discontinuité entre une partie où le nerf est immobilisé et celle où il est libre crée localement des contraintes à l'endroit de la transition, contraintes qui peuvent endommager les tissus nerveux.

Un autre inconvénient, également propre aux manchons de l'art antérieur, tient au fait que si l'on considère l'extrémité la plus intérieure du manchon 20 enroulé en spirale, le fait que cette extrémité soit enserrée par le reste du manchon a pour conséquence d'exercer des contraintes sur le nerf VN dans la région 22 située au voisinage de cette extrémité (contraintes schématisées par les flèches 24), qui ont pour effet de déformer le nerf de façon permanente, avec des effets potentiellement délétères.

Un autre inconvénient est lié à la création d'électrodes virtuelles telles qu'explicitées ci-dessous.

La stimulation neuronale est une activation artificielle de la fibre nerveuse, l'activation étant une stimulation électrique légère, notamment par application d'une brève impulsion de courant électrique sur la membrane de la fibre nerveuse appelé le potentiel d'action.

Toutefois, l'intérieur de la membrane du nerf n'est pas accessible et le courant à travers la membrane doit être obtenu par application d'un champ de potentiel externe.

Les lois d'Ohm et de Kirchhoff indiquent que le courant pénétrant la membrane (Im) est défini de la manière suivante :

$$Im \approx d(\sigma z \cdot d(Uz))/dz^2,$$

z étant le diamètre du nerf,
$\sigma z$ étant la conductivité électrique selon l'axe du nerf, et
Uz étant le potentiel appliqué sur la membrane.

Le courant membranaire externe active la membrane par dépolarisation alors que le courant membranaire interne inhibe l'activation ou la propagation des potentiels d'action par hyperpolarisation.

Le matériau de support des électrodes, en général un élastomère de silicone, présente une très faible conductivité comparée aux fluides et aux tissus biologiques entourant le manchon.

Ainsi, le courant généré par les électrodes le long du nerf va rencontrer un changement brusque de conductivité du matériau ($\sigma z$) aux deux extrémités du manchon. Il en résulte une génération de courants internes correspondant à une électrode virtuelle de type anode, ou de courants externes correspondant à une électrode virtuelle de type cathode, à travers la membrane. Ces électrodes virtuelles peuvent stimuler le nerf ou au contraire bloquer la transmission d'un potentiel d'action. Ces effets sont dans la plupart des cas une nuisance puisqu'ils ne peuvent être contrôlés.

Un autre inconvénient de ce dispositif de l'état de la technique est dû au fait que les anodes et cathodes virtuelles n'agissent pas uniquement sur le nerf mais également sur l'environnement du manchon entourant le nerf, à savoir les muscles, la peau ou d'autres nerfs provoquant pour le patient des effets indésirables.

Enfin, un tel manchon ayant une certaine rigidité, notamment sur les bords du manchon, peut endommager le nerf par la création d'une pression sur le nerf, tendant à contraindre et déformer le nerf. En outre, les bords du manchon peuvent exercer des forces de cisaillement sur le nerf. Ces différents inconvénients, ainsi que ceux exposés en introduction, peuvent être résolus par un manchon réalisé selon les enseignements de l'invention, illustré aux Figures 3 à 7.

La Figure 3 est une vue en plan du manchon selon l'invention, en configuration déroulée telle que ce manchon se présente au moment de sa fabrication c'est-à-dire dans une position initiale où la feuille formant le manchon est maintenue sous contrainte à l'état déployé et la Figure 5 est une vue du manchon conformément à l'invention dans sa configuration enroulée c'est-à-dire dans une position finale où la feuille formant le manchon est librement enroulée en spirale, notamment autour du nerf NV, présentant des extrémités évasées afin de minimiser toute contrainte dans la région comprise entre la partie du nerf enfermée à l'intérieur du manchon 26 et la partie libre du nerf au-delà du manchon.

Le manchon 26 selon l'invention comprend une feuille 26 en matériau élastiquement déformable. La feuille 26 est réalisée notamment à partir de deux feuilles d'élastomère 26a et 26 b (Figure 4) fixées ensemble, par exemple de silicone.

Selon un mode de réalisation, l'une des feuilles d'élastomère 26a et 26b est soumise préalablement à son assemblage avec l'autre feuille d'élastomère à une précontrainte d'étirement dans la direction $\Delta$, qui est dans cet exemple la direction de plus grande dimension de la feuille 26. Comme expliqué dans le US 4 602 624 A précité, cette technique permet de rendre le manchon auto-enroulable en spirale lorsque la feuille 26, après fabrication du manchon, ne sera plus soumise à aucune contrainte extérieure, conduisant à la configuration enroulée. De la sorte, la feuille 26 est précontrainte de manière à permettre son auto-enroulement depuis une position initiale où la feuille est maintenue sous contrainte à l'état déployé jusqu'à une position finale où la feuille est librement enroulée en spirale en formant un manchon autour de l'organe.

Selon un autre mode de réalisation, les deux feuilles d'élastomère 26a et 26b sont soumises préalablement à leur assemblage à une précontrainte d'étirement dans la direction $\Delta$, qui est dans cet exemple la direction de plus grande dimension de la feuille 26. Toutefois, l'une des feuilles d'élastomère est soumise à un précontrainte d'étirement plus forte que l'autre feuille d'élastomère.

Le silicone est choisi préférentiellement comme matériau de base pour le manchon implantable en raison de ses excellentes propriétés de biocompatibilité, tant au niveau de la biotolérance (l'implant n'induit pas de dommage à l'hôte : absence de toxicité et d'endommagement mécanique des tissus) que de sa biostabilité (l'implant résiste aux conditions induites par l'hôte).

Conformément à l'invention, afin de créer au moins une extrémité du manchon en une forme évasée, la feuille 26 dans la position initiale déployée, comprend au moins une zone de plus faible précontrainte à proximité du premier et/ou du second bord transversal sur les bords longitudinaux par rapport à une zone précontrainte située dans une région médiane de la feuille de sorte à créer au moins une extrémité évasée lorsque la feuille est librement enroulée en spirale.

Dans la position finale du manchon, c'est-à-dire enroulée, la feuille comprend au moins une zone ayant une contrainte à proximité du premier et/ou du second bord transversal sur les bords longitudinaux inférieure à la contrainte d'une zone située dans une région médiane de la feuille de sorte à créer au moins une extrémité évasée lorsque la feuille est librement enroulée en spirale.

Ainsi, lorsque le manchon est enroulé la zone ayant une contrainte inférieure présente un diamètre supérieur au diamètre de la zone ayant une contrainte supérieure, notamment située dans la région médiane du manchon.

Selon un mode de réalisation particulier, la feuille 26 porte, dans la région destinée à venir en contact avec le nerf vague après enroulement (la région située à gauche sur la Figure 3) un certain nombre d'électrodes 28 rapportées en surface de la feuille ou enfoncées dans l'épaisseur du matériau élastomère.

De manière alternative, les électrodes 28 sont positionnées entre les deux feuilles d'élastomères 26a et 26b. La feuille d'élastomère en contact avec le nerf vague après enroulement peut alors comprendre des ouvertures traversantes ou non, au niveau des électrodes.

Ces électrodes 28 sont reliées à des fils 30 destinés à être reliés au générateur d'impulsions 10. Dans l'exemple illustré sur la Figure 3, ces électrodes 28 sont distribuées de manière uniforme le long de l'axe d'enroulement du manchon 26 et elles sont reliées entre elles de manière à constituer une matrice de contacts de type quasi-tripôle (anode/cathode/anode ou l'inverse) reliés aux microcâbles 30 correspondants.

Pour réaliser le manchon de l'invention, il est possible d'utiliser des feuilles d'épaisseur relativement mince (de l'ordre de 100 $\mu$m), ce qui conduit à des manchons très souples, et donc très bien tolérés, sans compromis sur la facilité de pose et avec une transition très progressive entre le nerf et le manchon.

Le manchon est réalisé à partir de la feuille 26 qui présente une forme rectangulaire, avec un bord latéral intérieur 32 formant une première largeur (qui viendra à l'intérieur de la spirale après enroulement du manchon), un deuxième bord latéral extérieur 34 opposé formant une seconde largeur (qui viendra à l'extérieur du manchon après enroulement), et un premier bord transversal 36 réunissant des premières extrémités homologues du premier bord latéral 32 et du second bord latéral 34, et un second bord transversal 38 opposé réunissant des secondes extrémités homologues du premier bord latéral 32 et du second bord latéral 34. Comme illustré en Figure 3, les coins à angle droit de cette feuille rectangulaire peuvent être découpés (par estampage, découpe à la lame ou tout autre processus industriel adapté) de manière à éliminer les régions délimitées par les tirets 40, formant ainsi des bords en biseau 42, 44, 46 48. Ces biseaux forment par exemple un angle $\beta$ de 30° à 60° et s'étendent sur une largeur z de 10 à 25 % de la largeur totale l de la feuille 26.

De manière alternative, les coins à angle droit de cette feuille rectangulaire peuvent être arrondis.

Par ailleurs, dans le sens de l'épaisseur, l'extrémité 32 peut avoir un bord chanfreiné 50, le chanfrein étant tourné vers la face de la feuille qui sera appliquée contre le nerf. Ce chanfrein est incliné d'un angle $\gamma$ compris entre 20° et 45° par exemple.

Conformément à l'invention, la feuille 26, dans la position librement enroulée en spirale, comprend au moins une zone ayant une contrainte à proximité du premier et/ou du second bord transversal 36, 38 sur les bords longitudinaux inférieure à la contrainte d'une zone située dans une région médiane de la feuille de sorte à créer une ou deux extrémités évasées du manchon, comme illustré en Figure 5.

Le fait qu'une contrainte soit plus forte dans la partie médiane du manchon par rapport à l'un ou les deux bords longitudinaux implique que le manchon est fermement maintenu sur le nerf, et que les extrémités du manchon sont plus souples que la partie médiane. De la sorte, les extrémités évasées du manchon lorsqu'il est dans la position enroulée permettent d'une part d'éviter la création d'électrodes virtuelles et d'autre part, de créer un cisaillement du nerf au niveau de l'extrémité du manchon.

En effet, la forme évasée des extrémités du manchon permet de réduire la différence de conductivité aux extrémités du manchon, ce qui a pour conséquence de supprimer la génération non souhaitée d'électrodes virtuelles.

Comme illustré Figure 5, la zone de la feuille du manchon ayant une plus faible contrainte est localisée dans au moins une zone s'étendant jusqu'à 50 % de la largeur de la feuille à proximité du premier et/ou du second bord transversal, la largeur de la feuille étant définie par la distance entre le premier et le second bord transversal, en d'autres termes, la largeur correspond à la hauteur du manchon tel que représenté en Figure 5. Selon un mode de réalisation particulier, la zone de plus faible contrainte s'étend jusqu'à 30 % de la largeur de la feuille à proximité du premier et/ou du second bord transversal dans la position finale du manchon, c'est-à-dire enroulée.

Selon un autre mode de réalisation particulier, la zone de plus faible contrainte présente un gradient de contrainte décroissant sur la largeur de la zone, s'étendant de l'intérieur du manchon vers le bord transversal du manchon, de telle sorte que les contraintes sont inférieures en périphérie du manchon par rapport à la partie sensiblement médiane du manchon. En effet, afin de créer une forme évasée de manière progressive, la zone de plus faible contrainte, n'est pas formée sur toute sa largeur d'une même force de contrainte mais au contraire, une différence de contrainte est appliquée de manière décroissante de la zone proche de la partie médiane du manchon vers l'autre extrémité de la zone s'étendant vers le bord transversal de la feuille 26 de sorte à créer un gradient de contrainte sur ladite zone allant d'une contrainte plus forte vers la partie centrale du manchon pour aller avec une contrainte plus faible en périphérie de manchon.

Ce gradient de contrainte entre sensiblement la partie médiane du manchon et le bord périphérique du manchon peut être créé par l'utilisation de moyens d'étirement 71 positionnés à chaque extrémité de la feuille d'élastomère dans le sens de l'étirement à réaliser (comme illustré en Figure 7 détaillée ci-dessous).

Selon un mode de réalisation avantageux, la feuille 26 comprend deux zones de plus faible contrainte à proximité du premier et du second bord transversal de sorte à créer deux extrémités évasées lorsque la feuille est librement enroulée en spirale comme illustré Figure 5.

Bien entendu, l'invention s'applique avec un ou deux zones de plus faible contrainte situées sur l'un ou les deux bords transversaux de la feuille de manière à créer un manchon ayant une ou deux extrémités de forme évasées.

En référence aux Figures 6 à 8, on va maintenant décrire une méthode de fabrication d'un manchon pour sonde implantable selon l'invention.

La méthode de fabrication, dont les étapes successives sont schématisées Figure 6, débute par une étape E1 consistant à positionner une première feuille d'élastomère non étirée sur un support

L'étape E1 est suivie d'une étape E2 consistant à positionner une deuxième feuille d'élastomère dans un moyen d'étirement apte à étirer la feuille d'élastomère dans le sens longitudinal de l'étirement à réaliser.

La Figure 7 illustre les deux moyens d'étirement 71 fixés sur les deux extrémités de la deuxième feuille d'élastomère à étirer.

Selon le mode de réalisation illustré en Figure 7, les moyens d'étirement 71 sont de forme courbe du côté de la feuille afin de créer une précontrainte d'étirement décroissante s'étendant de la partie médiane des bords latéraux de la feuille d'élastomère vers les côtés transversaux de la feuille d'élastomère.

Comme illustré Figure 8, les moyens d'étirement courbes ont une forme circulaire du côté de la feuille d'élastomère ayant un rayon r créant une distance d entre les positions d'étirement dans la partie médiane de la feuille et au niveau des bords transversaux.

La distance d réduit la précontrainte créée sur les bords transversaux. En effet, la précontrainte réalisée au niveau de la partie médiane de la feuille élastomère peut être mesurée de la manière suivante :

$$(L_2 - L_1) / L_1 ,$$

$L_1$ étant la longueur de la feuille entre les deux supports d'étirement avant étirement, la longueur étant définie par la distance entre les deux supports d'étirement dans la partie médiane de la feuille d'élastomère et

$L_2$ étant la longueur de la feuille entre les deux supports d'étirement après étirement.

Et la précontrainte réalisée au niveau des bords transversaux de la feuille élastomère peut être mesurée de la manière suivante :

$$(L_2 - L_1) / (L_1 + d) ,$$

L1 étant la longueur de la feuille entre les deux supports d'étirement avant étirement, la longueur étant définie par la distance entre les deux supports d'étirement, et

L2 étant la longueur de la feuille entre les deux supports d'étirement après étirement.

Selon un exemple illustratif de ce mode de réalisation, un manchon selon l'invention ayant des extrémités évasées peut être obtenu en ayant une précontrainte lors de la fabrication de 70 % selon l'axe X au niveau de la partie médiane de la feuille élastomère, un rayon de courbure r des moyens d'étirement 71 entre 160 et 60 mm, ce qui aura pour conséquence de créer une distance d entre 5 et 15 mm.

L'étape E2 est suivie d'une étape E3 d'étirement de la deuxième feuille d'élastomère dans une direction Δ perpendiculaire à l'axe d'enroulement de la feuille.

Selon l'invention, il est réalisé un étirement plus faible dans une zone à proximité du premier et/ou du second bord transversal par rapport à une zone précontrainte située dans une région médiane de la feuille.

Puis l'étape E3 est suivie de l'étape E4 de fixation de la deuxième feuille d'élastomère étirée sur la première feuille d'élastomère non étirée. La fixation peut être réalisée par exemple par collage.

Selon un mode de réalisation particulier, les deux feuilles d'élastomère sont étirées différemment c'est-à-dire la première feuille d'élastomère subit un étirement et la seconde feuille d'élastomère subit un étirement plus élevé conformément à l'étape E3.

L'étape E4 est suivie d'une étape E5 de découpage du manchon à la taille souhaitée, notamment un découpage selon l'axe X afin de définir la longueur de la feuille formant le manchon.

Selon un mode de réalisation particulier, la zone de plus faible étirement est localisée dans au moins une zone s'étendant jusqu'à 50 % de la largeur de la feuille d'élastomère à proximité du premier et/ou du second bord transversal, la largeur de la feuille d'élastomère étant définie par la distance entre le premier et le second bord transversal de la feuille 26. Selon un mode de réalisation particulier, la zone de plus faible contrainte s'étend jusqu'à 20 % de la largeur de la feuille à proximité du premier et/ou du second bord transversal.

La méthode de fabrication d'un manchon décrite ci-dessus comprend en outre une étape de positionnement d'un certain nombre d'électrodes sur la face intérieure ou extérieure de l'une des deux feuilles d'élastomères ou dans l'épaisseur d'une des deux feuilles d'élastomère dans la région destinée à venir en contact avec le nerf vague après enroulement. Si les électrodes sont positionnées telles qu'elles ne sont pas en contact avec le nerf, alors la méthode comprend en outre une étape de réalisation d'ouvertures traversantes ou non à proximité des électrodes de sorte à améliorer la stimulation du nerf vague. Les ouvertures peuvent être réalisées par poinçonnage ou par découpe laser.

Les Figures 9 à 11 illustrent une variante de mise en oeuvre de la méthode de fabrication décrite ci-dessus.

Ici encore, la feuille 26 comprend avantageusement deux zones de plus faible contrainte à proximité du premier et du second bord transversal de sorte à créer deux extrémités évasées lorsque la feuille est librement enroulée en spirale comme illustré en Figure 5.

Comme illustré par exemple Figures 10 et 11, ces zones de plus faible contrainte sont les zones formées d'une seule feuille d'élastomère. Bien entendu, l'invention s'applique avec une ou deux zones de plus faible contrainte situées sur l'un ou les deux bords transversaux de la feuille de manière à créer un manchon ayant une ou deux extrémités de forme évasées. La Figure 9 illustre l'assemblage des deux feuilles d'élastomère, montrant la différence de largeur des deux feuilles d'élastomère. Cette différence de largeur permet la création d'un gradient de contrainte de sorte à obtenir un manchon conformément à la Figure 6. L'axe X représenté sur la Figure 9 illustre l'axe d'enroulement de la feuille obtenue.

Comme illustré par exemple Figures 10 et 11, afin de créer au moins une extrémité en forme évasée du manchon, la feuille 26 comprend au moins une zone formée d'une feuille d'élastomère à proximité du premier et/ou du second bord transversal sur les bords longitudinaux et une zone formée de la superposition des deux feuilles d'élastomère 26a et 26b situées dans une région médiane de la feuille, au moins une feuille d'élastomère de la superposition ayant subi un étirement avant son assemblage avec la seconde feuille d'élastomère.

De la sorte, il est créé au niveau de la jonction entre la zone comprenant une feuille d'élastomère et la zone ayant deux feuilles d'élastomère une différence de gradient de contrainte créant la forme évasée du manchon représentée Figure 5.

Ainsi, le manchon comprend dans sa position finale, c'est-à-dire enroulée, au moins une zone composée d'une seule feuille d'élastomère et une zone médiane composée d'au moins deux feuilles d'élastomères, la zone ou les zones composées d'une feuille d'élastomère ont une contrainte inférieure à la zone composée de deux feuilles d'élastomère. Le passage de la zone de la feuille 26 composée d'une feuille d'élastomère à la zone de la feuille 26 composée de deux feuilles d'élastomère crée une extrémité évasée lorsque la feuille est librement enroulée en spirale.

La Figure 10 illustre un assemblage de deux feuilles d'élastomère de largeurs différentes.

Conformément à l'invention, la première feuille d'élastomère et la seconde feuille d'élastomère sont de largeurs différentes, la largeur étant définie par la distance entre les bords latéraux desdites feuilles d'élastomère. En particulier, la feuille d'élastomère de plus faible largeur a une largeur comprise entre 60 et 80 % de la largeur de l'autre feuille d'élastomère. Du fait de la différence de largeur des deux feuilles d'élastomère, au moins un bord transversal 36, 38 est de plus faible épaisseur. Ainsi, il est créé une différence d'épaisseur sur le ou les bords transversaux de la feuille 26 de sorte à créer après enroulement, une forme évasée sur l'une ou les deux extrémités du manchon.

De plus, du fait de la présence d'une force de précontrainte sur une feuille d'élastomère et de l'absence de précontrainte sur l'autre feuille d'élastomère, la ou les zones situées sur les bords transversaux présentant une seule épaisseur, auront tendance à se déformer. Ainsi, lors de l'enroulement, cette ou ces zones vont créer la forme évasée d'une ou des deux extrémités du manchon montrée en Figure 5.

De par la forme évasée des extrémités du manchon, la génération d'électrodes virtuelles est supprimée. En outre, l'extrémité évasée étant souple, une telle extrémité n'endommagera pas l'organe entouré par le manchon, notamment l'organe à proximité des extrémités du manchon.

Selon un mode de réalisation particulier, l'étape de fixation consiste à fixer la feuille d'élastomère de plus faible largeur sensiblement centrée sur la largeur de la feuille d'élastomère la plus large.

De la sorte, il est créé un manchon présentant deux extrémités de forme évasée.

Comme illustré Figure 11, de manière alternative la feuille d'élastomère de plus faible largeur peut être positionnée de telle sorte à créer une feuille 26 présentant un bord transversal ayant une zone de plus faible épaisseur de largeur supérieure 73 à la zone de plus faible épaisseur présente sur le second bord transversal 72. Il est créé ainsi un manchon ayant deux extrémités évasées, toutefois la hauteur de la partie évasée est supérieure à l'une des extrémités par rapport à la seconde extrémité. Pour ce faire, la feuille d'élastomère de plus faible largeur est positionnée par exemple, sur la partie basse de la largeur de la feuille d'élastomère la plus large. Ce mode de réalisation est avantageux lorsque l'organe entouré par le manchon présente au niveau d'une extrémité du manchon un fort rayon de courbure. Dans un tel cas, la forme évasée de l'extrémité du manchon permet de ne pas blesser par cisaillement l'organe.

La première feuille d'élastomère peut être de largeur inférieure à la seconde feuille d'élastomère. Ainsi la feuille d'élastomère subissant le pré-étirement est de largeur supérieure à la feuille d'élastomère non étirée. Inversement, la seconde feuille d'élastomère peut être de largeur inférieure à la première feuille d'élastomère. Ainsi la feuille d'élastomère portant les électrodes a une largeur inférieure à la largeur de la feuille d'élastomère subissant l'étirement.

## Revendications

1. Une sonde implantable comprenant un manchon apte à être enroulé autour d'un organe de forme cylindrique allongée tel qu'un nerf (VN), ce manchon comprenant une feuille (26) en matériau élastiquement déformable portant au moins une électrode (28) de détection/stimulation,

   la feuille (26) étant précontrainte de manière à permettre son auto-enroulement depuis une position initiale où la feuille est maintenue sous contrainte à l'état déployé jusqu'à une position finale où la feuille est librement enroulée en spirale en formant un manchon autour de l'organe, la feuille (26) étant délimitée par un bord latéral extérieur (34) du manchon après enroulement, un bord latéral intérieur (32) du manchon après enroulement, et un premier bord transversal (36) réunissant des premières extrémités homologues du premier bord latéral et du second bord latéral, et un second bord transversal (38) opposé réunissant des secondes extrémités homologues du premier bord latéral et du second bord latéral, **caractérisée en ce que** dans la position finale du manchon, la feuille (26) comprend au moins une zone ayant une contrainte à proximité du premier et/ou du second bord transversal sur les bords longitudinaux inférieure à la contrainte d'une zone située dans une région médiane de la feuille de sorte à créer au moins une extrémité de la feuille évasée.

2. Sonde implantable selon la revendication 1, **caractérisée en ce que** la zone ayant une plus faible contrainte est localisée dans au moins une zone s'étendant jusqu'à 50 % de la largeur de la feuille à proximité du premier et/ou du second bord transversal, la largeur de la feuille étant définie par la distance entre le premier et le second bord transversal.

3. Sonde implantable selon la revendication 1, **caractérisée en ce que** la zone de plus faible contrainte présente un gradient de contrainte décroissant sur la largeur de la zone, s'étendant de l'intérieur du manchon vers le bord transversal du manchon.

4. Sonde implantable selon la revendication 1, **caractérisée en ce que** la zone ayant une plus faible contrainte est formée d'une feuille d'élastomère et la zone située en zone médiane comprend au moins deux feuilles d'élastomère.

5. Méthode de fabrication d'un manchon pour une sonde implantable, apte à être enroulé autour d'un organe de forme cylindrique allongée tel qu'un nerf (VN), ce manchon comprenant une feuille (26) en matériau élastiquement déformable portant au moins une électrode (28) de détection/stimulation, la feuille (26) étant précontrainte de manière à permettre son auto-enroulement depuis une position initiale où la feuille est maintenue sous contrainte à l'état déployé jusqu'à une position finale où la feuille est librement enroulée en spirale en formant un manchon autour de l'organe,

   la feuille (26) étant délimitée par un bord latéral extérieur (34) du manchon après enroulement, un bord latéral intérieur (32) du manchon après enroulement, et un premier bord transversal (36) réunissant des premières extrémités homologues du premier bord latéral et du second bord latéral, et un second bord transversal (38) opposé réunissant des secondes extrémités homologues du premier bord latéral et du second bord latéral, la feuille (26) étant formée d'au moins une première et une seconde feuilles d'élastomère (26a, 26 b),

**caractérisée en ce que** la méthode comprend :

- une étape d'étirement (E3) de la deuxième feuille d'élastomère dans une direction (Δ) perpendiculaire à l'axe d'enroulement de la feuille, un étirement plus faible étant réalisé dans une zone à proximité du premier et/ou du second bord transversal sur les bords longitudinaux par rapport à une zone précontrainte située dans une région médiane de la feuille, et
- une étape de fixation (E4) de la deuxième feuille d'élastomère étirée sur la première feuille d'élastomère.

6. Méthode selon la revendication 5, **caractérisée en ce que** la zone ayant une plus faible contrainte est localisée dans au moins une zone s'étendant jusqu'à 30 % de la largeur de la feuille d'élastomère à proximité du premier et/ou du second bord transversal, la largeur de la feuille d'élastomère étant définie par la distance entre le premier et le second bord transversal de la feuille (26).

7. Méthode selon la revendication 5, **caractérisée en ce que** la zone de plus faible contrainte présente un gradient de contrainte décroissant sur la largeur de la zone, s'étendant de l'extrémité de la zone la plus proche de la partie médiane de la feuille d'élastomère vers le bord transversal de la zone de plus faible précontrainte.

8. Méthode de fabrication d'un manchon pour une sonde implantable, apte à être enroulé autour d'un organe de forme cylindrique allongée tel qu'un nerf (VN), ce manchon comprenant une feuille (26) en matériau élastiquement déformable portant au moins une électrode (28) de détection/stimulation, la feuille (26) étant précontrainte de manière à permettre son auto-enroulement depuis une position initiale où la feuille est maintenue sous contrainte à l'état déployé jusqu'à une position finale où la feuille est librement enroulée en spirale en formant un manchon autour de l'organe,

la feuille (26) étant délimitée par un bord latéral extérieur (34) du manchon après enroulement, un bord latéral intérieur (32) du manchon après enroulement, et un premier bord transversal (36) réunissant des premières extrémités homologues du premier bord latéral et du second bord latéral, et un second bord transversal (38) opposé réunissant des secondes extrémités homologues du premier bord latéral et du second bord latéral,
la feuille (26) étant formée d'au moins une première et une seconde feuille d'élastomère (26a, 26 b),

**caractérisée en ce que** la méthode comprend :

- une étape d'étirement (E3) de la deuxième feuille d'élastomère dans une direction (Δ) perpendiculaire à l'axe d'enroulement de la feuille,
- une étape de fixation (E4) de la deuxième feuille d'élastomère étirée sur la première feuille d'élastomère,

la première feuille d'élastomère et la seconde feuille d'élastomère étant de largeurs différentes, la largeur étant définie par la distance entre les bords latéraux desdites feuilles d'élastomère.

9. Méthode selon la revendication 8, **caractérisée en ce que** l'étape de fixation consiste à fixer la feuille d'élastomère de plus faible largeur sensiblement centrée sur la largeur de la feuille d'élastomère la plus large.

10. Méthode selon la revendication 8, **caractérisée en ce que** la feuille d'élastomère de plus faible largeur a une largeur comprise entre 60 et 80 % de la largeur de l'autre feuille d'élastomère.

11. Méthode selon la revendication 8, **caractérisée en ce que** la première feuille d'élastomère est de largeur inférieure à la seconde feuille d'élastomère.

12. Méthode selon la revendication 8, **caractérisée en ce que** la seconde feuille d'élastomère est de largeur inférieure à la première feuille d'élastomère.

**Patentansprüche**

1. Implantierbare Sonde mit einer Hülse, die dazu bestimmt ist, um ein längliches zylindrisches Organ wie einen Nerv (VN) gewickelt zu werden, wobei diese Hülse eine Folie (26) aus elastisch verformbarem Material aufweist, die mindestens eine Detektions-/Stimulationselektrode (28) trägt, wobei die Folie (26) vorgespannt ist, um ihre Selbstaufrollung von einer Anfangsposition, in der die Folie im entfalteten Zustand unter Spannung gehalten wird, in eine Endposition zu ermöglichen, in der die Folie frei spiralförmig aufgerollt ist, wobei sie eine Hülse um das Organ bildet, wobei die Folie (26) durch einen äußeren Seitenrand (34) der Hülse nach dem Aufrollen eines inneren Seitenrandes (32) der Hülse nach dem Aufrollen begrenzt ist, und eine erste Querkante (36), die erste homologe Enden der ersten Seitenkante und der zweiten Seitenkante verbindet, und eine zweite, gegenüberliegende Querkante (38), die zweite homologe Enden der ersten Seitenkante und der zweiten Seitenkante verbindet, **dadurch ge-**

kennzeichnet, dass die Folie (26) in der Endposition der Hülse mindestens einen Bereich umfasst, der eine Spannung in der Nähe der ersten und/oder zweiten Querkante an den Längskanten aufweist, die geringer ist als die Spannung eines Bereichs in einem mittleren Bereich der Folie, um mindestens ein aufgeweitetes Ende der Folie zu erzeugen.

2. Implantierbare Sonde nach Anspruch 1, **dadurch gekennzeichnet, dass** sich der Bereich mit geringerer Spannung in mindestens einem Bereich befindet, der sich bis zu 50 % der Breite der Folie in der Nähe der ersten und/oder der zweiten Querkante erstreckt, wobei die Breite der Folie durch den Abstand zwischen der ersten und der zweiten Querkante definiert ist.

3. Implantierbare Sonde nach Anspruch 1, **dadurch gekennzeichnet, dass** der Bereich mit der geringsten Spannung einen abnehmenden Spannungsgradienten über die Breite des Bereichs aufweist, der sich von der Innenseite der Hülse in Richtung der Querkante der Hülse erstreckt.

4. Implantierbare Sonde nach Anspruch 1, **dadurch gekennzeichnet, dass** der Bereich mit der geringsten Spannung aus einer Elastomerplatte gebildet ist und der Bereich im mittleren Bereich aus mindestens zwei Elastomerplatten besteht.

5. Verfahren zur Herstellung einer Hülse für eine implantierbare Sonde, die dazu bestimmt ist, um ein längliches zylindrisches Organ wie einen Nerv (VN) gewickelt zu werden, wobei diese Hülse eine Folie (26) aus elastisch verformbarem Material umfasst, die mindestens eine Detektions-/Stimulationselektrode (28) trägt, wobei die Folie (26) so vorgespannt ist, dass sie sich von einer Anfangsposition, in der die Folie im entfalteten Zustand unter Spannung gehalten wird, bis zu einer Endposition, in der die Folie frei spiralförmig gewickelt ist, selbst aufrollen kann und so eine Hülse um den Nerv bildet, die Folie (26) durch eine äußere Seitenkante (34) der Hülse nach dem Aufwickeln, eine innere Seitenkante (32) der Hülse nach dem Aufwickeln und eine erste Querkante (36), die erste homologe Enden der ersten Seitenkante und der zweiten Seitenkante verbindet, und eine gegenüberliegende zweite Querkante (38), die homologe zweite Enden der ersten Seitenkante und der zweiten Seitenkante verbindet, begrenzt ist, wobei die Folie (26) aus mindestens einer ersten und einer zweiten Elastomerfolie (26a, 26b) gebildet ist, **dadurch gekennzeichnet, dass** das Verfahren umfasst:

- einen Schritt des Streckens (E3) der zweiten Elastomerfolie in einer Richtung (A) senkrecht zur Wickelachse der Folie, wobei eine geringere Streckung in einer Zone in der Nähe der ersten und/oder zweiten Querkante an den Längskanten in Bezug auf eine vorgespannte Zone, die sich in einem mittleren Bereich der Folie befindet, durchgeführt wird, und
- einen Schritt der Befestigung (E4) der zweiten Elastomerplatte, die auf die erste Elastomerplatte gespannt ist.

6. Verfahren nach Anspruch 5, **dadurch gekennzeichnet, dass** sich der Bereich geringerer Spannung in mindestens einem Bereich befindet, der sich bis zu 30 % der Breite der Elastomerplatte in der Nähe der ersten und/oder zweiten Querkante erstreckt, wobei die Breite der Elastomerplatte durch den Abstand zwischen der ersten und zweiten Querkante der Platte (26) definiert ist.

7. Verfahren nach Anspruch 5, **dadurch gekennzeichnet, dass** die Zone mit der geringsten Spannung einen abnehmenden Spannungsgradienten über die Breite der Zone aufweist, der sich von dem Ende der Zone, das dem mittleren Teil der Elastomerplatte am nächsten liegt, in Richtung der Querkante der Zone mit der geringsten Vorspannung erstreckt.

8. Verfahren zur Herstellung einer Hülse für eine implantierbare Sonde, die dazu bestimmt ist, um ein längliches zylindrisches Organ wie einen Nerv (VN) gewickelt zu werden, wobei diese Hülse eine Folie (26) aus elastisch verformbarem Material umfasst, die mindestens eine Detektions-/Stimulationselektrode (28) trägt, wobei die Folie (26) so vorgespannt ist, dass sie sich von einer Anfangsposition, in der die Folie im entfalteten Zustand unter Spannung gehalten wird, bis zu einer Endposition, in der die Folie frei spiralförmig gewickelt ist und eine Hülse um die Elektrode bildet, selbst aufrollen kann, wobei die Platte (26) durch eine äußere Seitenkante (34) der Hülse nach dem Wickeln, eine innere Seitenkante (32) der Hülse nach dem Walzen und eine erste Querkante (36), die erste homologe Enden der ersten Seitenkante und der zweiten Seitenkante verbindet, und eine gegenüberliegende zweite Querkante (38), die homologe zweite Enden der ersten Seitenkante und der zweiten Seitenkante verbindet, begrenzt ist, wobei die Platte (26) aus mindestens einer ersten und einer zweiten Elastomerplatte (26a, 26b) gebildet ist, **dadurch gekennzeichnet, dass** das Verfahren umfasst:

- einen Schritt des Streckens (E3) der zweiten Elastomerfolie in einer Richtung (Ä), die senkrecht zur Wickelachse der Bahn verläuft,
- einen Schritt der Befestigung (E4) der zweiten Elastomerplatte, die auf die erste Elastomerplatte gespannt ist,

die erste Elastomerplatte und die zweite Elastomerplatte sind unterschiedlich breit, wobei die Breite durch den Abstand zwischen den Seitenkanten der Elastomerplatten definiert ist.

9. Verfahren nach Anspruch 8, **dadurch gekennzeichnet, dass** der Befestigungsschritt das Befestigen der schmaleren Elastomerplatte im Wesentlichen mittig auf der Breite der breiteren Elastomerplatte umfasst.

10. Verfahren nach Anspruch 8, **dadurch gekennzeichnet, dass** die schmalere Elastomerfolie eine Breite zwischen 60 und 80 % der Breite der anderen Elastomerfolie aufweist.

11. Verfahren nach Anspruch 8, **dadurch gekennzeichnet, dass** die erste Elastomerfolie eine geringere Breite aufweist als die zweite Elastomerfolie.

12. Verfahren nach Anspruch 8, **dadurch gekennzeichnet, dass** die zweite Elastomerplatte eine geringere Breite als die erste Elastomerplatte aufweist.

**Claims**

1. An implantable probe comprising a sleeve adapted to be wound around an elongated cylindrical organ such as a nerve (VN), this sleeve comprising a sheet (26) of elastically deformable material carrying at least one detection/ stimulation electrode (28), the sheet (26) being pre-stressed so as to allow its self-rolling from an initial position in which the sheet is held under tension in the deployed state to a final position in which the sheet is freely rolled up in a spiral, forming a sleeve around the member, the sheet (26) being delimited by an outer lateral edge (34) of the sleeve after rolling up an inner side edge (32) of the sleeve after winding, and a first transverse edge (36) joining first homologous ends of the first side edge and the second side edge, and a second opposite transverse edge (38) joining second homologous ends of the first side edge and the second side edge, **characterised in that** in the final position of the sleeve, the sheet (26) comprises at least one area having a stress near the first and/or second transverse edge on the longitudinal edges less than the stress of an area in a medial region of the sheet so as to create at least one flared end of the sheet.

2. The implantable probe according to claim 1, **characterised in that** the area with lower stress is located in at least one area extending up to 50% of the width of the foil in the vicinity of the first and/or the second transverse edge, the width of the foil being defined by the distance between the first and the second transverse edge.

3. An implantable probe according to claim 1, **characterised in that** the area of lowest stress has a decreasing stress gradient across the width of the area, extending from the inside of the sleeve towards the transverse edge of the sleeve.

4. An implantable probe according to claim 1, **characterised in that** the area with the lowest stress is formed of an elastomer sheet and the area in the middle area comprises at least two elastomer sheets.

5. A method of manufacturing a sleeve for an implantable probe, adapted to be wound around an elongated cylindrical organ such as a nerve (VN), this sleeve comprising a sheet (26) of elastically deformable material carrying at least one detection/stimulation electrode (28), the sheet (26) being pre-stressed so as to allow its self-rolling from an initial position where the sheet is held under stress in the deployed state to a final position where the sheet is freely wound in a spiral, forming a sleeve around the nerve,

the sheet (26) being bounded by an outer side edge (34) of the sleeve after winding, an inner side edge (32) of the sleeve after winding, and a first transverse edge (36) joining first homologous ends of the first side edge and the second side edge and an opposite second transverse edge (38) joining homologous second ends of the first side edge and the second side edge, the sheet (26) being formed of at least first and second elastomeric sheets (26a, 26b), **characterised in that** the method comprises :

- a step of stretching (E3) the second elastomer sheet in a direction (A) perpendicular to the winding axis of the sheet, a lower stretch being performed in a zone near the first and/or second transverse edge on the longitudinal edges with respect to a pre-stressed zone located in a middle region of the sheet, and
- a step of fixing (E4) the second elastomer sheet stretched on the first elastomer sheet.

6. A method according to claim 5, **characterised in that** the area of lower stress is located in at least one area extending up to 30% of the width of the elastomer sheet in the vicinity of the first and/or second transverse edge, the width of the elastomer sheet being defined by the distance between the first and second transverse edge of the sheet (26).

7. A method according to claim 5, **characterised in that** the zone of lowest stress has a decreasing stress gradient across the width of the zone, extending from the end of the zone closest to the middle part of the elastomer sheet towards the transverse edge of the zone of lowest pre-stress.

8.  A method of manufacturing a sleeve for an implantable probe, adapted to be wound around an elongated cylindrical organ such as a nerve (VN), this sleeve comprising a sheet (26) of elastically deformable material carrying at least one detection/stimulation electrode (28), the sheet (26) being pre-stressed so as to allow its self-rolling from an initial position where the sheet is held under stress in the deployed state to a final position where the sheet is freely wound in a spiral forming a sleeve around the electrode,
    the sheet (26) being bounded by an outer side edge (34) of the sleeve after winding, an inner side edge (32) of the sleeve after rolling, and a first transverse edge (36) joining first homologous ends of the first side edge and the second side edge and an opposite second transverse edge (38) joining homologous second ends of the first side edge and the second side edge, the sheet (26) being formed of at least first and second elastomeric sheets (26a, 26b), **characterised in that** the method comprises :

    - a step of stretching (E3) the second elastomer sheet in a direction (À) perpendicular to the winding axis of the sheet,
    - a step of fixing (E4) the second elastomer sheet stretched on the first elastomer sheet,

    the first elastomer sheet and the second elastomer sheet being of different widths, the width being defined by the distance between the side edges of said elastomer sheets.

9.  The method of claim 8, **characterised in that** the fixing step comprises fixing the narrower elastomer sheet substantially centred on the width of the wider elastomer sheet.

10. A method according to claim 8, **characterised in that** the narrower elastomer sheet has a width between 60 and 80% of the width of the other elastomer sheet.

11. A method according to claim 8, **characterised in that** the first elastomer sheet is of lesser width than the second elastomer sheet.

12. A method according to claim 8, **characterised in that** the second elastomer sheet is of smaller width than the first elastomer sheet.

Fig.1

Fig.2

Fig.3

Fig.4

# Fig.5

26

# Fig.6

| Positionnement d'une première feuille d'élastomère non étirée sur un support | E1 |

↓

| Positionnement d'une deuxième feuille d'élastomère dans un moyen d'étirement | E2 |

↓

| Etirement de la deuxième feuille d'élastomère | E3 |

↓

| Fixation de la deuxième feuille d'élastomère sur la première feuille d'élastomère | E4 |

↓

| Découpage du manchon | E5 |

Fig.7

Fig.8

Fig.9

Fig.10

Fig.11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4602624 A
- US 5251634 A
- WO 2014106023 A
- US 2008004673 A
- EP 2959938 A